# EUROPEAN PATENT APPLICATION

(11) **EP 4 703 391 A1**
(43) Date of publication of application: **04.03.2026**
(21) Application number: 23935425.1
(22) Date of filing: 14.07.2023
(51) Int. Cl.: C08B 37/00, A23K 20/163, A23L 33/125, A61K 8/73, A61K 31/739, A61K 35/74, A61P 3/06, A61P 3/08, A61P 3/10, A61P 17/00, A61Q 19/00, A61Q 19/10

(54) **LIPOPOLYSACCHARIDE, METHOD FOR PRODUCING LIPOPOLYSACCHARIDE, AND LIPOPOLYSACCHARIDE FORMULATION**

(30) Priority: 24.04.2023 JP 2023071020
(71) Applicant: SOMA Gen-Ichiro, Setagaya-ku Tokyo 158-0084 (JP); Biomedical Research Group Inc., 158-0084 Tokyo (JP)
(72) Inventor: INAGAWA, Hiroyuki, Takamatsu-shi, Kagawa 761-8062 (JP); KOHCHI, Chie, Takamatsu-shi, Kagawa 761-8075 (JP); SOMA, Gen-Ichiro, Tokyo 158-0084 (JP)
(74) Representative: Engelhard, Markus
(86) International application number: PCT/JP2023/026043
(87) International publication number: WO 2024/224651

(57) **Abstract**

With an object of producing a low molecular mass lipopolysaccharide of high purity at low cost by deposited bacteria that produces the low molecular mass lipopolysaccharide of high purity, a lipopolysaccharide obtained from deposited bacteria obtained by causing a change in the genetic information of conventional Pantoea agglomerans, a lipopolysaccharide production method of obtaining the lipopolysaccharide from the deposited bacteria, and a lipopolysaccharide composition that is a pharmaceutical, veterinary drug, quasi drug, cosmetic, food, functional food, feedstuff, fertilizer, or bath agent, etc., in which the lipopolysaccharide is contained are provided.

## Description

### [Technical Field]

The present invention relates to a lipopolysaccharide that is an immunostimulatory substance safe even for addition to a pharmaceutical, quasi drug, cosmetic, food, functional food, feedstuff, fertilizer, bath agent, etc., for animals, including humans, and plants, a method for producing the lipopolysaccharide, and a lipopolysaccharide composition containing the lipopolysaccharide.

### [Background Art]

A lipopolysaccharide (may also be referred to hereinafter as "LPS") is a composite compound constituted of a lipid and a saccharide and is present in the outer membrane that surrounds the peptidoglycan of cell walls of gram-negative bacteria. As a basic structure, it is constituted of the three components of lipid A that has a distinctive lipid, an oligosaccharide called core polysaccharide that is covalently bonded thereto, and further, an O-antigen polysaccharide with diverse structures depending on bacteria. Although generally with the O-antigen polysaccharide, several saccharides make up a specific structure that is connected repeatedly as a unit, there is diversity in the number of repetitions and therefore the molecular mass is nonuniform (NPL 1).

LPS exhibits diverse pharmacological actions. Examples include an effect of preventing onset of diabetes, an effect of suppressing dyslipidemia, a suppression effect against atopic dermatitis, an adjuvant effect of increasing effects of vaccines, etc. (NPL 2).

We have found that LPS (LPSp) of Pantoea agglomerans isolated from wheat exhibits high effects when administered orally (NPL 1).

Upon analyzing the LPSp, we have found that there are a low molecular mass type (LMM) and a high molecular mass type (HMM). We then introduced a gel filtration method using deoxycholic acid and succeeded in separating the LPSp into LMM-LPSp and HMM-LPSp. The LMM-LPSp showed almost 10 times higher tumor necrosis factor (TNF) inducing activity, an indicator of immune activation, compared to the original LPS after intravenous administration (PTL 1 and NPL 3).

However, so far, although the gel filtration method using deoxycholic acid can be implemented in a laboratory scale, it is difficult to industrialize, is extremely costly, and thus not practical. To solve this problem, we have developed a culture method with which more LMM-LPSp is contained than HMM-LPSp (PTL 2).

### [Citation List]

### [Patent Literature]

[PTL 1] JP 4043533 B2
[PTL 2] JP 2022-160938 A
[PTL 3] JP 5511112 B2

### [Non Patent Literature]

[NPL 1] I. Lerouge et al., "O-antigen structural variation: mechanisms and possible roles in animal/plant-microbe interactions," FEMS Microbiology Reviews, 2002.03, 26(1), pp. 17-47.
[NPL 2] C. Kohchi et al., "Applications of lipopolysaccharide derived from Pantoea agglomerans (IP-PA1) for health care based on macrophage network theory," Journal of Bioscience and Bioengineering, 2006.12, 102(6), pp. 485-496.
[NPL 3] T. Kadowaki et al., "Induction of nitric oxide production in RAW 264.7 cells under serum-free conditions by O-antigen polysaccharide of lipopolysaccharide," ANTICANCER RESEARCH, 2013.07, 33(7), pp. 2875-9.

### [Summary of Invention]

### [Technical Problem]

However, even with the culture method of PTL 2, a non-negligible amount of HMM-LPSp is still contained. Thus, if bacteria that produces LMM-LPSp of high purity exists, it becomes possible to easily produce LMM-LPSp of high purity and an ideal LPS can be produced at low cost.

### [Solution to Problem]

A lipopolysaccharide of the present invention is characterized in being obtained from bacteria of accession number NITE BP-03562.

Also, a lipopolysaccharide production method of the present invention is characterized in obtaining a lipopolysaccharide from bacteria of accession number NITE BP-03562.

Also, a lipopolysaccharide composition of the present invention is characterized in that the lipopolysaccharide is contained therein.

The lipopolysaccharide composition is preferably a pharmaceutical, veterinary drug, quasi drug, cosmetic, food, functional food, feedstuff, fertilizer, or bath agent.

### [Advantageous Effects of Invention]

With the lipopolysaccharide of the present invention, due to being obtained from bacteria (deposited bacteria) obtained by causing a change in the genetic information of conventional Pantoea agglomerans, that of ideal low molecular mass can be obtained without using a special gel filtration method, etc. Also, bioactivity of the lipopolysaccharide of the present invention is higher than those of conventional lipopolysaccharides. The lipopolysaccharide of the present invention is a novel lipopolysaccharide that differs in molecular mass and saccharide chain composition from conventional lipopolysaccharides. Further, since the lipopolysaccharide of the present invention is released by heating from the deposited bacteria, an adjustment method for animal feed raw material, food raw material, or cosmetic raw material that omits a purifying process used up to now can be used and adjustment can thus be performed at low cost.

### [Brief Description of Drawings]

[FIG. 1] FIG. 1 is a diagram showing an electrophoretogram that shows a molecular mass of the lipopolysaccharide of the present invention.
[FIG. 2] FIG. 2 is a diagram showing an NO production activity of the lipopolysaccharide of the present invention.

### [Description of Embodiments]

Preferred embodiments of the present invention shall now be described in detail.

### [Example 1]

### 1. Preparation of Pantoea agglomerans strain IG1-47

### (1-1) Acquisition of mutant strain by mutagen treatment of Pantoea agglomerans

A portion of a colony of Pantoea agglomerans (strain IG1) was scraped, inoculated on an LB (Luria-Bertani) agar medium, and cultured overnight inside a constant temperature reservoir at 35°C. One of the colonies was cultured in a sterilized LB medium (50 mL scale in a baffled flask) for 16 hours (35°C, 170 rpm). Thereafter, the culture solution was centrifuged (3000 rpm × 5 min) to collect bacterial bodies that was then suspended in a 50 mM potassium phosphate solution (pH 6.0). The bacterial count was prepared to 1 × 10^7 cells/mL and 1 mL thereof was dispensed into a 1.5 mL tube. To the tube, an N-methyl-N'-nitro-N-nitrosoguanidine (MNNG) solution was added as a mutagen to a final concentration of 100 µg/mL and mixed gently. Thereafter, temperature was controlled at 37°C for 0.5, 1, 1.5, 2, 3, and 4 hours. After each duration, the bacteria were collected by centrifugation, and washed twice with the 50 mM potassium phosphate solution (pH 6.0) (the operation is centrifuging after suspending, discarding the supernatant, and resuspending). The cell suspension was plated on an LB agar medium, incubated overnight at 37°C, and the number of colonies was counted the next day (Table 1).

With colonies of MNNG treatments of 3 hours and 4 hours, which had 99.9% mortality, the shapes of colonies were observed visually. Strains that formed larger colonies, whiter colonies, and colonies with less gloss were preferentially selected over untreated colonies. Consequently, (1) 108 large, white, less glossy colonies, (2) 61 yellow, smooth colonies, (3) 3 yellow, rough colonies, (4) 6 pale yellow, smooth colonies, (5) 36 milky white, smooth colonies, and (6) 2 milky white, rough colonies were obtained as candidate mutant strains.

**[Table 1]**

| Table 1. Mutagen treatment | | | | |
|---|---|---|---|---|
| Treatment | Treatment duration | Plate count | Death rate | Remarks |
| | (hr) | (/mL) | (%) | |
| Untreated | | 7,050,000 | 0.0 | |
| MNNG 100 µg/mL | 0.5 | 412,000 | 94.16 | |
| | 1 | 309, 000 | 95.62 | |
| | 1.5 | 222,500 | 96.84 | |
| | 2 | 142, 000 | 97.99 | |
| | 3 | 8,000 | 99.89 | Selected colonies. |
| | 4 | 5,000 | 99.93 | Selected colonies. |

### (1-2) Screening of high LPS content strains from isolated strains

108 strains (No. 1 to No. 108) were cultured on an LB plate and bacterial bodies were collected. Upon measuring the wet bacterial body weight, 1 mL of a 10 mg/mL aqueous suspension was prepared and after heat treatment at 90°C for 20 minutes, centrifugation was performed and the supernatant was obtained as a hot water extract solution. An LPS amount contained in this solution was measured as a Limulus value (value obtained by a Limulus test; value in terms of LPS from control standard endotoxin Escherichia coli UKT-B) by a toxinometer (FUJIFILM Wako Pure Chemical Corporation). Five strains of high LPS content (Nos. 11, 25, 32, 41, and 47) were selected as candidate strains.

The untreated (parent strain) and the five candidate strains were cultured in an LB medium (50 mL scale in a baffled flask) for 16 hours (35°C, 170 rpm) (n=3) . After culturing, bacterial bodies were collected, the wet bacterial body weights were measured, 10 mg/mL aqueous suspensions were prepared, and the hot water extract solutions thereof was obtained. The LPS amount contained in each hot water extract was determined as the Limulus value.

Consequently, the LPS contents per wet bacterial body weight of the candidate strains No. 11 and No. 47 were 20 mg/g or more, which were higher than the parental strain's average of 11.72 ±1.28 mg/g and the candidate strains No. 11 and No. 47 were both found to be excellent in LPS production.

A crushed bacterial body can be cited as a method for adjusting an LPS (PTL 3). Since this uses a fermentation culture solution (with which LPS are contained in both the bacterial bodies and the culture solution), it is desirable to evaluate an LPS production amount per culture solution. The parent strain and the candidate strains No. 11 and No. 47 were thus cultured and upon comparing the LPS production amounts per 42.5 mL of the culture solution, No. 47 was found to be highest in LPS production amount of the bacterial bodies and also highest in total LPS production amount combining the bacterial bodies and the culture supernatant (Table 2).

**[Table 2]**

| Table 2. Bacterial cell, supernatant, and total LPS production amounts | | | |
|---|---|---|---|
| Strain | LPS production amount (mg/42.5 mL culture solution) | | |
| | Bacterial bodies | Supernatant | Total |
| Parent strain | 4.08 | 5.90 | 9.98 |
| No. 11 | 3.67 | 8.82 | 12.49 |
| No. 47 | 5.97 | 6.89 | 12.86 |

### (1-3) NO production activity inducibility of candidate strains

The bioactivity of LPS was evaluated in terms of induction activity of nitric oxide (NO), which is a cell signaling molecule in macrophages and is also an antiviral and antibacterial substance.

To RAW 264.7 cells (1.6 × 10^5 cells/well), which are peripheral blood mononuclear cells were cultured for 24 hours with a culture solution containing a sample of two-times concentration. As an NO production amount, a concentration of nitrite, which is a metabolite thereof, was measured with a Griess reagent. A concentration of an LPS (LPSp: Funakoshi, mac0001) derived from Pantoea agglomerans was expressed as dry weight and the LPS concentration of each sample was compared by the Limulus measurement.

Results: The NO production of the strain No. 47 was found to be higher than those of the strain No. 11 and the parent strain (Table 3).

**[Table 3]**

| Table 3. NO production amounts | | | |
|---|---|---|---|
| LPSp concentration (ng/mL) | Nitrite concentration (µM) | | |
| | Parent strain (average ± SD) | No. 11 (average ± SD) | No. 47 (average ± SD) |
| 0 | 0.28±0.1 | 0.28±0.17 | 0.28±0.17 |
| 0.01 | 0.22±0.12 | 0.22±0.13 | 0.29±0.22 |
| 0.1 | 0.28±0.12 | 0.29±0.13 | 0.36±0.19 |
| 1 | 6.61±0.81 | 5.90±0.46 | 8.66±0.84* |

| | | | |
|---|---|---|---|
| *: Significantly higher than the parent strain and No. 11 by Tukey's multiple comparison test (n = 4; P < 0.001). | | | |

As a result of screening, the No. 47 strain was selected as a high LPS content strain candidate based on the LPS collecting amount and NO production activity, named as IG1-47, and deposited (Biomaterials depositary: NITE Patent Microorganisms Depositary, National Institute of Technology and Evaluation; Address: #122, 2-5-8 Kazusakamatari, Kisarazu-shi, Chiba 292-0818, Japan; Accession number: NITE BP-03562; Date of deposit: November 25, 2021).

### 2. Evaluation of properties of LPS (IG1-47 LPSp) of the Pantoea agglomerans strain IG1-47

### (2-1) Culturing of Pantoea agglomerans strain IG1-47

The parent strain of Pantoea agglomerans (strain IG1) and strain IG1-47 were cultured with the LB medium for 16 hours (35°C, 170 rpm) at the 50 mL scale using a baffled flask. After culturing, bacterial bodies were collected by centrifugation of the culture solutions (3.5 krpm, 30 minutes). Supernatants were collected in separate containers. The wet bacterial body weights of the collected bacteria were measured. An average value and standard deviation of the wet bacterial body collecting amounts of three examples of strain IG1-47 were 273.4 mg ± 3.6 mg. An aqueous suspension of 10 mg/mL was prepared from the wet bacterial bodies. 0.5 mL of the suspension was heated at 90°C for 30 minutes. After the heat treatment, ultrasonication (10 minutes) and stirring using a vortex mixer were performed. The suspension was diluted with water and the Limulus value was measured using the toxinometer (FUJIFILM Wako Pure Chemical Corporation).

### (2-2) Purification of lipopolysaccharide from bacterial bodies of Pantoea agglomerans strain IG1-47

A portion of a colony of strain IG1-47 was scraped, inoculated on an LB agar medium, and cultured overnight inside a constant temperature reservoir at 35°C. One colony was added to 10 mL of a sterilized LB medium and precultured under a shaking condition of 170 rpm at 35°C. 0.05 mL of the preculture solution were placed in a 250 mL baffled flask containing 50 mL and shaking cultured at 35°C overnight. After culturing, the culture solution was transferred into a 50 mL centrifuge tube, centrifuged (KUBOTA Model 5220 Tabletop Centrifuge) at 3500 rpm, and 276.1 mg of wet bacterial bodies were collected. Purification of liposaccharide from the wet bacterial bodies of strain IG1-47 was performed in accordance with a method by Westphal et al. That is, distilled water was added to 276.1 mg of the wet bacterial bodies to adjust to 100 mg/mL of wet bacterial bodies. The bacteria were suspended, a same volume of 90% phenol was added to the liquid, and stirred at 65°C to 70°C for 15 minutes. Thereafter, the liquid was cooled to 4°C and centrifugation at 3500 rpm was performed. 1 mL of an aqueous layer that is an upper layer was collected in a separate container, distilled water of the same amount as the collected aqueous layer was added to the remaining phenol layer and intermediate layer, stirred again at 65°C to 70°C for 10 minutes, and the lipopolysaccharide was reextracted. Thereafter, the liquid was cooled to 4°C and centrifugation was performed. Upon combining 2 mL of the aqueous layer of the second time with the aqueous layer of the first time, distilled water was added to dilute by 10 times and low molecular mass substances including phenol were removed using a centrifugal ultrafiltration filter with a molecular mass cutoff of 10,000. 3 mL of the ultrafiltration inner liquid was further treated with a nuclease (Benzonase) (20 U/ml). The cleaved nucleic acids were removed by ultrafiltration. The inner liquid was treated with a protease (Proteinase K) (200 µg/ml), followed by phenol extraction, and thereafter, phenol was removed from the aqueous layer using a centrifugal ultrafiltration filter with a molecular mass cutoff of 10,000. 4 mL of the inner liquid was collected. This concentrated liquid was used as a purified lipopolysaccharide (IG1-47 LPSp) solution of Pantoea agglomerans strain IG1-47. This solution was freeze-dried and thedry weight was measured. The dry weight of the purified lipopolysaccharide was 2.0 mg.

### (2-3) Measurement of molecular mass of lipopolysaccharide IG1-47 LPSp

Molecular masses of IG1-47 LPSp (0.1 µg/lane) and chromatographically purified LMM-LPSp (0.1 µg/lane) were analyzed by sodium dodecyl sulfate-polyacrylamide gel electrophoresis (gel of 20% concentration; SDS-PAGE) using tricine. After the electrophoresing, silver staining by a silver stain kit (Cat 291-50301, Silver Stain II Kit, FUJIFILM Wako Pure Chemical Corporation) was performed for the purpose of visualizing the molecules of lipopolysaccharide. Since LPS, which is glycolipid, differs in behavior from protein molecular mass markers that are used ordinarily, glycolipids of known molecular mass size were used as molecular mass markers (Salmonella minnesota Rb2 LPS (molecular mass: 3,878 Da) (0.3 µg/lane) and Salmonella minnesota Rd2 LPS (molecular mass: 2,777 Da) (0.3 µg/lane)).

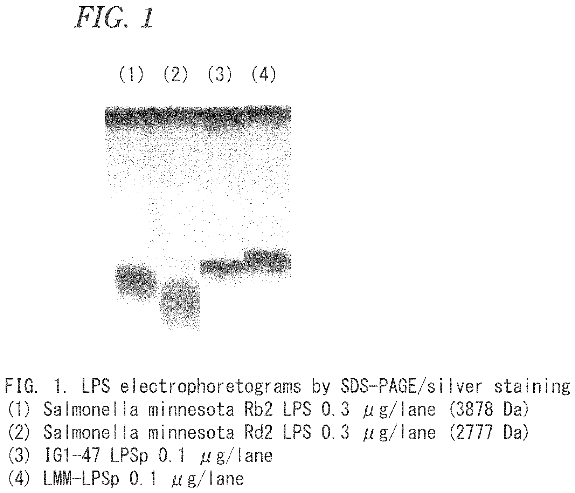

The results are shown in FIG. 1. IG1-47 LPSp of lane (3) showed a single band. Two bands were observed for the chromatographically purified parent strain LPS of lane (4) .

The band position of IG1-47 LPSp was slightly lower than the lower molecular mass band of the two bands of the chromatographically purified LMM-LPSp. Although an accurate molecular mass is not known, it was estimated to be approximately 4,000 Da.

### (2-4) Measurement of composition of lipopolysaccharide IG1-47 LPSp

10 mg of purified IG1-47 LPSp were weighed out on an electronic balance and added to a conical tube of 15 mL volume. Distilled water for injection was added to prepare a 2.0 mg/mL aqueous solution of IG1-47 LPSp.

### (2-4-1) Nucleic acid content measurement

For nucleic acid concentration measurement, 50 µL of 2.0 mg/mL IG1-47 LPSp aqueous solution were added to 950 µL of distilled water to make 100 µL/mL and a spectrum in a range of 200 nm to 340 nm was measured by a plate reader. A value obtained by subtracting an absorbance value at 320 nm from an absorbance value at 260 nm was multiplied by 50 µg/mL to determine the nucleic acid content. A peak was not observed at the absorption wavelength of 260 nm. The nucleic acid content estimated from the value obtained by subtracting the absorbance value at 320 nm from the absorbance value at 260 nm was 5.6 %. Since a peak was not observed at 260 nm, it was inferred that nucleic acids are fewer than the calculated value and since absorbance due to turbidity of IG1-47 LPSp is included, the estimated nucleic acid content was estimated to be not more than 5.6% (Table 4).

### (2-4-2) Protein content measurement

A protein content was measured by Lowry's method.

Bovine serum albumin was used as a standard for measuring protein content and the protein content in IG1-47 LPSp was measured to be 0.75% in terms of bovine serum albumin (Table 4).

### (2-4-3) Neutral sugar content measurement (phenol-sulfuric acid method)

A neutral sugar content was measured using the phenol-sulfuric acid method with glucose as a standard.

The measured value was 40.6%. That of the chromatographically purified LMM-LPSp, which was measured at the same time, was 34.0% (Table 4).

**[Table 4]**

| Table 4. Nucleic acid, protein, and neutral sugar contents of IG1-47 LPSp and chromatographically purified LMM-LPSp | | | |
|---|---|---|---|
| LPS | Nucleic acid (%) | Protein (%) | Neutral sugar (%) |
| No. 47 LPS Lot. 2101011 | <5.6 | 0.75 | 40.6 |
| Chromatographically purified LMM-LPSp Lot. 160708 | <0.22 | 0.24 | 34.0 |

### (2-4-4) Monosaccharide composition analysis (thermolysis method)

A monosaccharide composition was analyzed. After hydrolysis, it was analyzed by HPLC as ABEE derivative.

As a result, five types of sugars were measured from IG1-47 LPSp (Table 5). Five types of sugars were also measured from LMM-LPSp (Table 5).

A comparison of ratios of respective sugars (with GlcNAc set to 2) of IG1-47 LPSp and LMM-LPSp showed that those of glucose (Glc) and rhamnose (Rha) were respectively higher by approximately 1 in IG1-47 LPSp.

From these results, it was found that IG1-47 LPSp and LMM-LPSp differ in the monosaccharide composition ratios.

**[Table 5]**

| Table 5. Monosaccharide analysis of IG1-47 LPSp | | |
|---|---|---|
| Constituent saccharide | IG1-47 LPSp | LMM-LPSp |
| GalA | 3.79 | 4.25 |
| Gal | 1.34 | 1.22 |
| Glc | 3.19 | 1.99 |
| GlcNAc* | 2.00 | 2.00 |
| Rha | 1.24 | 0.19 |

| | | |
|---|---|---|
| *: Relative ratios when that of GlcNAc is set to 2 are shown. | | |

### (2-4-5) Measurement of immunostimulatory action 1 (NO production from RAW 264.7 cells)

Nitric oxide (NO) production from cells was measured in the cultured mouse macrophage cell line RAW 264.7 after addition of LPS using concentration of nitrite, which is an NO metabolite, in the culture solution as an indicator. RAW 264.7 was purchased from ATCC (No. TIB-71). LPSp (LPS of parent strain, mac0001) was used as a control. The RAW 264.7 cells were collected by pipetting from a culturing flask and the cell concentration was adjusted to 1.6 × 10^6 cells/ml with a culture solution (RPMI1640 medium containing 10% fetal bovine serum, 100 U/ml penicillin and 100 µg/ml streptomycin). 100 µL of the cell suspension (1.6 × 10^5 cells/100 µl) were transferred to each well of a 96-well flat-bottom plate and used in the test 3 hours later. IG1-47 LPSp was adjusted to 2000 ng/ml. Further, five 10-hold stepwise dilutions were made to prepare samples for addition to wells containing the cells. LPSp and the chromatographically purified LMM-LPSp were also tested at the same time. 100 µL of the respective samples were added to respective wells of the 96-well flat-bottom plate with the cells. It was cultured in a 5% carbon dioxide gas incubator for 24 hours at 37°C and after the culture, 100 µL of the supernatant was collected in another 96-well plate. The amount of nitrite, which is a metabolite of the nitric oxide, in the culture solution was measured using the Griess reagent according to usual method.

### [Measurement results]

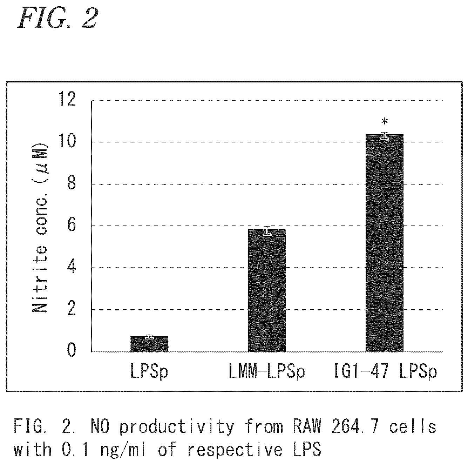

IG1-47 LPSp showed significantly higher NO production activity compared to the chromatographically purified LMM-LPSp and LPSp (FIG. 2).

### (2-4-6) Measurement of immunostimulatory action 2 (TNFα production in THP-1 cells)

TNFα production after addition of LPS to THP-1, a cultured cell line of human monocytic leukemia cells, was measured by ELISA. THP-1 (JCRB0112.1) was purchased from JCRB Cell Bank. LPSp (LPS of parent strain, mac0001) and the chromatographically purified LMM-LPSp were used as controls.

THP-1 cells were collected by pipetting from a culturing flask and the cell concentration was adjusted to 8 × 10^5 cells/ml with a culture solution (RPMI1640 medium containing 10% fetal bovine serum and containing 100 U/ml penicillin and 100 µg/ml streptomycin). 100 µL of the cell suspension (8 × 10^4 cells/100 µl) were transferred to each well of a 96-well flat-bottom plate. IG1-47 LPSp was adjusted to 2000 ng/ml. Further, five 10-hold stepwise dilutiona were made to prepare samples for addition to wells containing the cells. LPSp and the chromatographically purified LMM-LPSp were also tested at the same time. 100 µL of the respective samples were added to respective wells of the 96-well flat-bottom plate with the cells. It was cultured in a 5% carbon dioxide gas incubator for 24 hour at 37°C and after the culture, the supernatant was collected in plastic tubes. A TNFα concentration in the culture solution was measured according to a measurement method in a kit for TNFα.

### [Measurement results]

IG1-47 LPSp showed significantly higher TNFα production, as much as 5-fold at a dose of 0.1 ng/mL compared to the chromatographically purified LMM-LPSp (Table 6).

**[Table 6]**

| Table 6. TNFα production amounts of respective LPS (0.1 ng/ml) | |
|---|---|
| Sample | TNFα (pg/ml ± SD) |
| LPSp | 0.1±0.0 |
| LMM-LPSp | 2.0±2.1 |
| IG1-47 LPSp* | 10.1±2.6 |

| | |
|---|---|
| *: The TNFα production amount of IG1-47 LPSp showed a significant difference (P < 0.05) compared to LMM-LPSp and LPSp. | |

As described above, it was clarified that IG1-47 LPSp is structurally different from the conventional LMM-LPSp, has high innate immune activation capability, and can be produced at low cost.

The obtained lipopolysaccharide is beneficial for composition in pharmaceuticals, veterinary drugs, quasi drugs, cosmetics, foods, functional foods, feedstuffs, fertilizers, and bath agents, etc.

The disclosure of Japanese Patent Application No. 2023-071020 filed on April 24, 2023 that includes a description, claims, and drawings is incorporated herein by reference in its entirety.

All publications, patents, and patent applications cited in this Description are incorporated herein by reference in their entirety.

## Claims

1. A lipopolysaccharide obtained from a bacterium having accession number NITE BP-03562.

2. A lipopolysaccharide production method wherein a lipopolysaccharide is obtained from a bacterium having accession number NITE BP-03562.

3. A lipopolysaccharide composition contains the lipopolysaccharide according to Claim 1.

4. The lipopolysaccharide composition according to Claim 3 that is selected from a pharmaceutical, veterinary drug, quasi drug, cosmetic, food, functional food, feedstuff, fertilizer, or bath agent.
